# EUROPEAN PATENT APPLICATION

(11) **EP 4 603 107 A1**
(43) Date of publication of application: **20.08.2025**
(21) Application number: 24157839.2
(22) Date of filing: 15.02.2024
(51) Int. Cl.: A61K 47/54, A61P 35/00

(54) **BIOORTHOGONAL, ANILINIUM BASED CLEAVABLE TETRAZINES**

(71) Applicant: University of Copenhagen, 1165 Copenhagen K (DK)
(72) Inventor: HERTH, Matthias Manfred, 21121 Malmö (SE); POULIE, Christian Bernard Matthijs, 21837 Bunkeflostrand (SE); STAUDT, Markus, 2200 Copenhagen N (DK)
(74) Representative: Budde Schou A/S

(57) **Abstract**

The present invention provides compounds of formula I for bioorthogonal cleavage reactions so called click-to-release reactions, a kit comprising compounds of formula I and use of compounds of formula I. The compounds of formula I are tetrazines comprising an anilinium moiety and further comprising a payload entity to be released upon reaction with a dienophile. The present compounds of formula I with the anilinium moiety provide surprisingly improved release reaction rate with sufficient chemical stability. The compounds can be used for drug delivery, prodrug activation, modification of molecules under physiological conditions, solid phase synthesis, biomolecule interactions, and modification of surfaces.

## Description

### Field of the Invention

The present invention is within the field of bioorthogonal cleavage reactions so called click-to-release reactions. The compounds involved in such reactions will release an entity, such a drug, upon an inverse-electron demand Diels-Alder (IEDDA) click reaction between a diene, such as a tetrazine and a dienophile, such as *trans-*cyclooctene (TCO) at a specific target site. The compounds can for instance be used for drug delivery, prodrug activation, modification of molecules under physiological conditions, solid phase synthesis, biomolecule interactions, and modification of surfaces.

### Background

Bioorthogonal cleavage reactions, often referred to as "click-to-release" reactions, represent a powerful and versatile tool in the field of chemical biology. These reactions enable the controlled release of specific entities, such as drugs or bioactive molecules, particularly within biological systems. The cornerstone of this approach lies in the utilization of inverse-electron demand Diels-Alder (IEDDA) click chemistry, where a diene (typically tetrazine) reacts with a dienophile (commonly TCO) to form a stable covalent adduct.

The bioorthogonal nature of these cleavage reactions is paramount for their application in complex biological environments. This term signifies that the reactions occur selectively and efficiently within a biological system without interfering with native biochemical processes. This selectivity ensures minimal off-target effects, making bioorthogonal cleavage reactions an invaluable tool for precise and controlled delivery of entities such as therapeutic agents.

The compounds involved in these reactions are meticulously designed to undergo the IEDDA click reaction at specific target sites. This level of control allows for tailored drug delivery strategies, prodrug activation, and site-specific modifications of molecules under physiological conditions. Moreover, bioorthogonal cleavage reactions find extensive utility in solid-phase synthesis, enabling the rapid assembly of complex molecular architectures with high purity and efficiency.

The integration of these reactions into biological systems has far-reaching implications. They have been successfully employed in diverse applications, including probing biomolecule interactions, modifying surfaces for bioconjugation, and enhancing the functionality of therapeutic agents. The ability to precisely release bioactive entities in a spatiotemporally controlled manner has opened up new avenues in drug discovery, personalized medicine, and chemical biology research.

Some of the notable applications include treatment of diseases such as cancer, neurological disorders, infectious diseases, cardiovascular diseases, autoimmune disorders, metabolic disorders and rare diseases. In cancer therapy bioorthogonal cleavage reactions have been explored for targeted drug delivery. For instance, systems have developed where cytotoxic drugs are conjugated to cleavable linkers. These linkers release the active drug selectively in cancer cells upon bioorthogonal reaction with specific biomolecules.

Some of the strategies applied relate on prodrug activation wherein designed prodrugs are inactive until activated by a bioorthogonal reaction. This approach enables the controlled release of active therapeutic agents at specific sites within the body. This has been investigated for various conditions, including cancer and infectious diseases.

Different tetrazines and dienophiles have been used in bioorthogonal cleavage reactions. For example, 1,2,4,5-tetrazines are highly reactive and are often used in bioorthogonal cleavage reactions. They can rapidly react with TCOs to form a stable dihydropyrazine adduct. It is particularly useful for applications where rapid reaction kinetics are desired. The dienophile is often a trans-cyclooctene (TCO). The basic structure of TCO consists of an eight-membered ring containing one double bond in the trans-configuration. Substituted cyclooctene derivatives with different functional groups (e.g., alkyl, aryl, or polar groups) attached to the cyclooctene ring have been employed to modulate the reactivity and compatibility of TCOs in specific applications. Also, bicyclononyne (BCN), a strained cyclooctyne derivative that exhibits exceptionally high reactivity with tetrazines has found utility in various bioorthogonal applications.

While bioorthogonal cleavage reactions offer exciting possibilities, for example within drug delivery, there are several challenges and limitations associated with this technology such as reaction kinetics, biological compatibility, selectivity and specificity, delivery efficiency, in vivo stability, immunogenicity and toxicity. In relation to reaction kinetics, the rate of the click reaction between tetrazines and TCOs can vary depending on the specific structures and substituents. In some cases, reaction kinetics may not be fast enough for certain applications, potentially leading to suboptimal release profiles. Accordingly, effort has been applied for designing tetrazine derivatives with enhanced reactivity.

Tetrazines previously developed for click-to-release reactions comprise either a carbamate moiety, an ester moiety or an ether moiety. For example, WO2018/004338 discloses a tetrazine comprising a carbamate moiety conjugated to a construct of interest. Upon cycloaddition of the tetrazine with compatible dienophile, the construct is released from the tetrazine. Wang et al., Org. Lett. 2022, 5293-5297 discloses the bioorthogonal cleavage of physiologically stable methylene tetrazines bearing an ether or ester linkage in the presence of trans-cyclooctene.

The compounds of formula I provided herein are tetrazines comprising an anilinium moiety and further comprising a payload entity to be released upon reaction with a dienophile. The present compounds of formula I with the anilinium moiety provide surprisingly improved release reaction rate with sufficient chemical stability.

### Summary of the invention:

The present invention provides compounds of formula I: 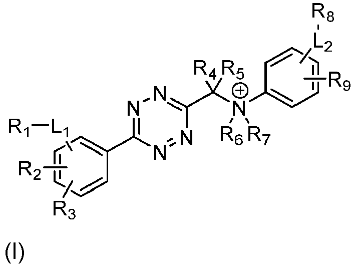 and salts thereof, wherein:
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙ NHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of: 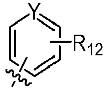
wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity.

In addition, the present invention relates to:
- A kit comprising the compound according to formula I and a dienophile,
- Compound of formula I for use as a medicament, and for use in the treatment of specific diseases such as cancer,
- Use of a compound of formula I in ex vivo click-to-release reactions for the release of a aniline with a payload in a chemical, biological or physiological environment

### Brief description of the drawings

Figure 1: Schematic drawing of the synthesis of a compound according to Formula (I)
Figure 2: Table showing release properties t½ (min) and percentage of various R₁ and R₂ groups after click-reaction between ten different tetrazines and five different dienophiles.

### Definitions

**Anilinium moiety** is a primary arylamine in which a quaternary nitrogen group is substituted for one of the benzene hydrogens.

The terms **targeting vector** and **pretargeting vector** are used interchangeable in the context of the present invention. The terms refer to a vector directed at a specific target. "Pre"-targeting refers to the way the targeting vector is used in for example pretargeted imaging and therapy comprising two entities, i.e. a "first" entity and a "second" entity to be combined at the target site. The "first" entity comprises the pretargeting vector and is exposed to the target prior to the "second" entity.

**"First entity"** and **"second entity"** refers in the context of the present invention to a diene of formula I and a dienophile with Diels-Alder activity complementary to the diene of formula I. The first entity comprises the pretargeting vector whereas the second entity comprises a payload that will be released shortly after the first and second entities reacts via an inverse-electron demand Diels-Alder (IEDDA) click reaction followed by a bioorthogonal cleavage reaction.

A **kit** is in the context of the present invention a combination of a diene according to the present invention and a dienophile with Diels-Alder activity complementary to the diene that are provided or sold together either in the same package or in separate packages with directions to be used together in a click-to-release reaction.

A **payload** is in the context of the present invention any entity that provides a desired effect on the target, such as a therapeutic agent.

A **target** is in the context of the present invention any entity of interest such as a molecule, which is present in or on an organism, tissue or cell. Targets include cell surface targets such as receptors, glycoproteins, glycans, carbohydrates; structural proteins; extracellular targets; intracellular targets and pathogens such as viruses, bacteria, fungi, yeast or parts thereof.

### Detailed description of the invention

The compounds of formula I provided here are tetrazines comprising an anilinium moiety and moreover comprising a payload entity to be released upon reaction with a dienophile, and further optionally comprising a targeting entity. The compounds of formula I may be in free form or in a salt form, such as in a pharmaceutically acceptable salt form.

The present tetrazines of formula I with the anilinium moiety provides improved release reaction rate of the payload upon click-to-release reaction with a dienophile with Diels-Alder activity complementary to the tetrazine of formula I. The present compound of formula I can be used for many purposes, one being within the field of medicaments and/or diagnostic agents for example targeted drug conjugates, prodrug activation in vivo, another being for applications in chemistry, ex vivo, and in vitro uses.

This invention is based on the tetrazine-ligation reaction. The "first" entity comprising the targeting vector can be either the tetrazine of formula I or the dienophile with Diels-Alder activity complementary to the tetrazine of formula I. This "first" entity comprising the targeting vector is administered to the subject comprising the target. After sufficient accumulation time, the "second" entity is administered. This "second" entity is a tetrazine of formula I, or a dienophile having complementary inverse electron demand Diels-Alder cycloaddition reactivity to the "first" entity, respectively. Within short, the tetrazine of formula I and the dienophile with Diels-Alder activity complementary to the tetrazine of formula I will make a bioorthogonal IEDDA-based reaction to provide a dihydropyridazine which is followed by a spontaneous immolative cascade rearrangement whereby the payload is released from the dihydropyridazine at the target site. The dienophile could be a cyclic or linear dienophile, preferably a *trans*-cyclooctene. In some embodiments, the dienophile, such as TCO, is conjugated to a pretargeting vector, with high specificity and selectivity for the target side. The pretargeting vector is for example an antibody, a nanobody, a polymer, a nanomedicine, a cell, a protein, a peptide, a carbohydrate, a monosaccharide, polysaccharide, an oligonucleotide or a small molecule with specificity for a certain target. In other embodiments, the pretargeting vector is comprised by the tetrazine of formula I instead of being comprised by the TCO. Thus, in some embodiments, the dienophile comprises the targeting vector and then the tetrazine of formula I need not comprise a targeting vector. In other embodiments, the tetrazine of formula I comprises the targeting entity and then, the dienophile need not comprise a targeting vector. Optionally, both the tetrazine of formula I and the dienophile could comprise a targeting vector.

After sufficient accumulation time in embodiments where the dienophile comprises the targeting vector and wherein the target is exposed to this dienophile-targeting vector conjugate prior to the tetrazine of formula I, the tetrazine of formula I comprising a payload, optionally a masked payload, such as a drug or a fluorophore, is then administrated and within a few minutes the tetrazine is covalently bound to the pretargeting vector. Followed by a fast (<15 min) spontaneous immolative cascade rearrangement, the payload, such as a parent drug, is released solely at the target site. This results in higher cytotoxic concentrations of the payload, such as a parent drug, at the target site and significantly reduced systemic concentrations. Therefore, this invention will simultaneously increase the therapeutic effectiveness, as well as lower any unwanted toxic side effects of the parent cytotoxic drug.

The tetrazines of the invention are compounds of formula I: 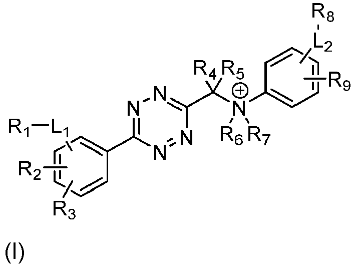 and salts thereof, wherein:
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

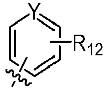

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity.

As shown herein, it was surprisingly found that the presence of the aniline moiety in compounds according to formula I significantly increases the second order rate constant of the inverse electron demand Diels-Alder cycloaddition click-reaction with a dienophile with Diels-Alder activity complementary to the compound of formula I compared to previously known tetrazines for similar purposes. A fast reaction time is crucial for many practical purposes for use of compounds according to formula I. For example, it improves patient compliance by reducing the treatment time and/or imaging time, and provides for the use of less stable payloads such as isotopes with rapid decay and payloads that are easily decomposed.

It was moreover surprisingly found that compounds according to formula I wherein the targeting vector or substituent is linked to a phenyl i.e. in their capacity of being phenyl tetrazines, the stability of the compounds are significantly improved compared with otherwise similar tetrazine compounds wherein a targeting vector is directly linked to the tetrazine.

The targeting vector / pretargeting vector can be any vector capable of binding to a desired target. In some embodiments, the compound according to formula I comprises a targeting vector / pretargeting vector. In other embodiments the compound of formula I does not comprise a targeting vector but is instead used in a reaction wherein the compound of formula I is reacted with a dienophile with Diels-Alder activity complementary to compound I and comprising a targeting vector / pretargeting vector.

The targeting vector (R₁ in compounds of formula I when R₁ is not a substituent) may target any suitable target such as a target within the human or animal body or on a pathogen or parasite. The targets are typically cellular components, receptors, intracellular structures, enzymes, DNA, RNA, viral particles, macrophages, antibodies, proteins, carbohydrates, monosaccharides, polysaccharides, cytokines, hormones, and steroids. Accordingly, when R₁ is a targeting vector, it is typically an antibody, derivatives or fragments of an antibody, a protein, a peptide, a carbohydrate, a monosaccharide, a polysaccharide, a oligonucleotide, a living cell, or a nanoparticle.

In a preferred embodiment, the compound according to formula I is a compound wherein R₁ is a targeting entity selected from: an antibody or a fragment thereof, a nanobody, a polymer, a nanomedicine, a cell, a protein, a peptide, a carbohydrate, a monosaccharide, a polysaccharide, an oligonucleotide, or a small molecule.

The compound according to formula I comprises a payload, R₈, to be released upon reaction with a dienophile with Diels-Alder activity complementary to compound I. The payload, R₈, can be any suitable payload, that provides a desired effect on the target, such as a therapeutic agent. The payload entity can accordingly be selected from very different compounds such as small organic molecule drugs, steroids, lipids, proteins, aptamers, oligopeptides, oligonucleotides, oligosaccharides, peptides, peptoids, amino acids, nucleotides, polynucleotides, nucleosides, DNA, RNA, toxins, glycans, hormones, cytotoxins, antiproliferative/antitumor agents, antiviral agents, antibiotics, cytokines, anti-inflammatory agents, antihypertensive agents, chemo pharmaceuticals, radiopharmaceuticals, anti-viral agents, immunosuppressants, immunostimulants enzyme inhibitors, neurotoxins, channel blockers, modulators of cell-extracellular matrix interactions, cell growth inhibitors, anti-adhesion molecules, inhibitors of DNA, RNA or protein synthesis, anti-inflammatory agents.

In a preferred embodiment, the payload, R₈, is selected from: a chemotherapeutic agent, an anti-angiogenic agent, a cytotoxic agent, an immunomodulatory agent, a hormone, an antibiotic, an anti-viral agent, an anti-inflammatory agent, or a cell-cycle suppressor.

In other preferred embodiments, the payload, R₈, is selected from: a radionuclide, an auristatin, a maytansine, a maytansinoid, a calicheamicin, a dactinomycine, a duocarmycin, camptothecin, tubulysin M, a cryptophycin, a pyrrolobenzodiazepine, methotrexate, methopterin, dichloromethotrexate, 5-fluorouracil, a DNA minor groove binder, 6-mercaptopurine, cytosine arabinoside, melphalan, leurosine, leurosideine, actinomycin, an anthracycline, a mitomycin, aminopterin, tallysomycin, podophyllotoxin, vinblastine, vincristine, vindesine, taxol, taxotere retinoic acid, butyric acid, NS-acetyl spermidine, staurosporin, colchicine, camptothecin, esperamicin, and an ene-diyne.

Each of the groups R₁ and R₈ may be linked to the compound of formula I directly or via a linker.

In one embodiment, R₁ is linked to the compound of formula I directly, and R₈ is linked to the compound of formula I directly.

In another embodiment, R₁ is linked to the compound of formula I directly, and R₈ is linked to the compound of formula I via a linker.

In another embodiment, R₁ is linked to the compound of formula I via a linker, and R₈ is linked to the compound of formula I via a linker.

In another embodiment, R₁ is linked to the compound of formula I via a linker, and R₈ is linked to the compound of formula I directly.

The linkers, when present, are selected independently from each other. In preferred embodiments the compound according to formula I comprises at least one of L₁ and

L₂ and L₃ is independently selected from -(CH₂CH₂O)ₘ-, where m is an integer from 1-10.

Compounds of formula I further comprises one or more of the groups R₂, R₃ and R₉. These groups are selected independently of each other. In preferred embodiments R₂, R₃ and R₉ are independently selected from: methyl, ethyl, OH, OCH₃, or OCH₂CH₃.

Compounds of formula I further comprises the groups R₄, and R₅. These groups are selected independently of each other. In preferred embodiments R₄, and R₅ are independently selected from: H, Methyl, Ethyl, or Phenyl.

In another preferred embodiment, the compound according to formula I is a compound wherein L₃ within at least one of L₁ and L₂ is independently selected from - (CH₂CH₂O)ₘ-, where m is an integer from 1-10; and wherein R₂, R₃ and R₉ are independently selected from: methyl, ethyl, OH, OCH₃, or OCH₂CH₃; and wherein R₄ and R₅ are independently selected from: H, Methyl, Ethyl, or Phenyl.

The compound of formula I may be in the free form or in a salt form. In some embodiments wherein the aniline comprising the payload is to be released in a living organism, such as a human, the salt is preferably a pharmaceutically acceptable salt.

The present invention also relates to a kit comprising at least two entities wherein one entity is a compound of formula I or a pharmaceutically acceptable salt thereof. The other entity is a dienophile with Diels-Alder activity complementary to the compound of formula I. Optionally, the kit further comprises instructions for use of the kit.

The entities of the kit may be provided in a single package or packed individually.

For example, the compound of formula I and the dienophile with Diels-Alder activity complementary to the compound of formula I may be provided in a single package or in individual packages or in individual subpackages comprised by an outer package combining the subpackages. The optional instructions for use may be provided in one of the packages or independently from the packages of the kit by the provider of the kit.

When using the kit, the first entity being the entity comprising the targeting vector is brought into contact with the target first. For example, if the kit is used in the treatment of a disease in a human, the entity comprising the targeting vector is administered first. Depending on the time required for the targeting vector to bind to the target, the second entity is administered subsequently. When the two entities are brought into contact at the target site, the bioorthogonal click-to-release reaction couple the two entities followed by release of the payload at the target site.

In order for the kit to be functional, at least one of the entities must comprise a targeting vector. Compounds according to formula I may or may not comprise a targeting vector. If the compound of formula I comprises a targeting vector, then the dienophile with Diels-Alder activity complementary to the compound of formula I need not comprise a targeting vector. If, on the other hand, the compound according to formula does not comprise a targeting vector, then it is necessary that the dienophile with Diels-Alder activity complementary to the compound of formula I, comprises a targeting vector.

Thus, the kit according to the present invention comprises a compound according to formula I and a dienophile with Diels-Alder activity complementary to the compound of formula I, with the proviso that when R₁ in the compound of formula I is a substituent selected from H, Methyl, Ethyl, Propyl, COOH, CONH₂, CONHCH₃, NH₂, NHCH₃, N(CH₃)₂, then the dienophile with Diels-Alder activity complementary to the compound of formula I comprises a targeting vector.

Any dienophile with Diels-Alder activity complementary to the selected compound of formula I provided in the kit may be applicable.

In a preferred embodiment, the kit comprises a dienophile with Diels-Alder activity complementary to compound I wherein the dienophile has a structure according to Formula II 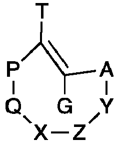 wherein A,Y, Z, X, Q, P each independently are selected from the group consisting of CR^{a}2, C=CR^{a}2, C=O, S, S=O, SO₂, O, NR^{b} and SiR^{c}₂, provided that A and P are not O or S and that NR^{b} if present in A or P, is part of an amide moiety, with at most three of A,Y, Z, X, Q and P being selected from the group consisting of C=CR^{a}₂ and C=O, wherein two or more R moieties together may form a ring or rings, and with the proviso that no sets consisting of adjacent atoms are present selected from the group consisting of O-O, O-NR^{b}, S-NR^{b} O-S, O-S(O), O-S(O)₂, and S-S, and such that Si is only adjacent to CR^{a}₂ or O;

T, G each independently denotes H, or a substituent selected from the group consisting of alkyl, OR', NR'R', F, Cl, Br, or I, with R' being alkyl or aryl;
each R^{a} as above-indicated can independently be selected from the group consisting of H, (hetero)alkyl, aryl, heterocycle, carbocycle, F, Cl, Br, I, CF₃, CF₂-R', NO₂, OR', SR', CN, C(=O)R', S(=O)R', S(=O)₂R", S(=O)₂OR', OS(=O)₂R", PO₃R'₂, Si-R"₃, Si-OR"₃, B(OR')₂,S(=O)₂NR'₂, NR'S(=O)₂R", C(=O)NR'₂, C(=S)NR'₂, C(=NR')NR'₂,NR'₂, NR"₃⁺,NR'C(=O)R', NR'C(=S)R', NR'C(=O)OR', NR'C(=S)OR', NR'C(=O)SR', NR'C(=S)SR',NR'C(=O)NR'₂, NR'C(=S)NR' ₂, wherein each R' is independently selected from the group consisting of H, (hetero)alkyl, aryl, carbocycle, heterocycle, and each R" is independently selected from the group consisting of (hetero)alkyl, aryl, carbocycle, heterocycle, wherein all (hetero)alkyl, aryl, carbocycle, heterocycle moieties comprised in R^{a} can be unsubstituted or substituted, and wherein two R' or R" groups can together form a ring;
each R^{b} is independently selected from the group consisting of H, (hetero)alkyl, aryl, heterocycle, carbocycle, OR', C(=O)R', C(=O)O-R', C(=O)S-R', C(=S)O-R', C(=S)S-R', C(=O)NR'₂, C(=S)NR'₂, C(=NR')NR'₂, NR'₂, preferably such that no sets consisting of adjacent atoms are present selected from the group consisting of -N-N-, -N-O-, -N-S-, -N-Si- and -N-P-, and wherein each R' is independently selected from the group consisting of H, (hetero)alkyl, aryl, carbocycle, heterocycle, wherein all (hetero)alkyl, aryl, carbocycle, heterocycle moieties comprised in R^{b} can be unsubstituted or substituted, and wherein two R' groups can together form a ring;
each R^{c} as above indicated is independently selected from the group consisting of H, alkyl, aryl, O-alkyl, O-aryl, OH;
wherein two or more R^{a,b,c} moieties together may form a ring;
wherein, optionally, one or more R^{a,b,c} moieties may be bound to one or more of the group consisting of Targeting vector T^{T}, Spacer S^{P}, chelator, albumin-binding moiety, polymersome, micelle, particle, resin, gel, surface, bead, membrane translocation moiety, radioisotope, and fluorescent moiety;
wherein, optionally said Targeting agent T^{T}, Spacer S^{P}, chelator, albumin-binding moiety, polymersome, micelle, particle, resin, gel, surface, bead, membrane translocation moiety, radioisotope, and fluorescent moiety, is bound to more than one dienophile moiety.

Compounds of formula I and salts thereof can be used for various purposes wherein it is desired to bring a payload into close contact with a specific target or wherein use of click-to-release is normally applied, such as in the treatment of different diseases.

Thus, one aspect of the invention is compounds of formula I: 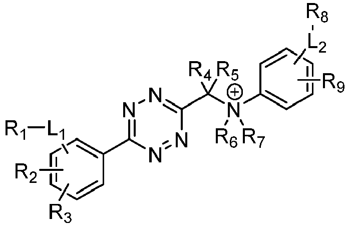 and pharmaceutically acceptable salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

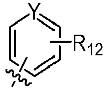

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; for use as a medicament.

In a preferred embodiment, the compound of formula I is to be administered in combination with a dienophile with Diels-Alder activity complementary to compound I for use as a medicament, thus, providing compounds of formula I: 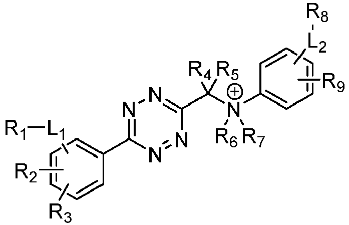 and pharmaceutically acceptable salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

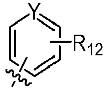

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; wherein the compound of formula I or a pharmaceutically acceptable salt thereof, is to be administered in combination with a Diels-Alder activity complementary to the compound of formula I, for use as a medicament.

A preferred embodiment is compounds of formula I: 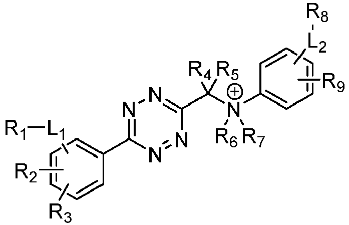 and pharmaceutically acceptable salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

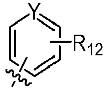

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; for use in the treatment of cancer.

In a preferred embodiment, the compound of formula I is to be administered in combination with a dienophile with Diels-Alder activity complementary to compound I for use in the treatment of cancer, thus, providing compounds of formula I: 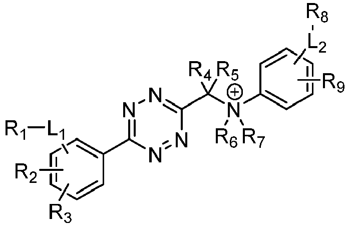 and pharmaceutically acceptable salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

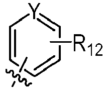

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; wherein the compound of formula I or a pharmaceutically acceptable salt thereof, is to be administered in combination with a Diels-Alder activity complementary to the compound of formula I, for use in the treatment of cancer.

Accordingly, a preferred embodiment is a compound according formula I: 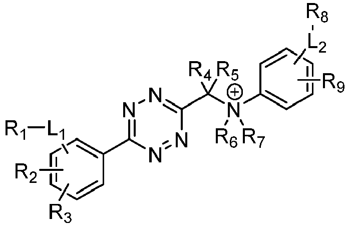 and pharmaceutically acceptable salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

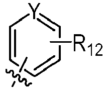

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; wherein the payload entity R₈ is an anti-cancer agent.

In another aspect, here is provided a method of treatment comprising:
- administering of a compound of formula I: 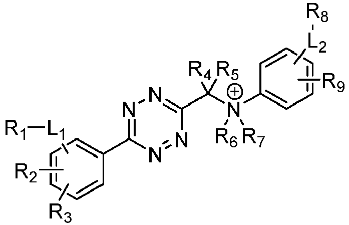 or a pharmaceutically acceptable salt thereof, wherein
   L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
   R₁ is a targeting vector, or a substituent selected from: H, Methyl, Ethyl, Propyl, COOH, CONH₂, CONHCH₃, NH₂, NHCH₃, or N(CH₃)₂;
   R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
   R₄ and R₅ are independently selected from: H, Methyl, Ethyl, CF₃, COOH, C₁-C₁₀, CONH₂, or Ar₁
   wherein Ar₁ is selected from the group consisting of:

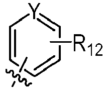

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity; to a subject in need thereof, and

- administering to the same subject a dienophile with Diels-Alder activity complementary to compound I.

In a preferred embodiment, the method is for the treatment of cancer in a human subject.

The compounds according to formula I and salts thereof, can also be used a chemical tool for release of a payload in ex vivo environments such as in a chemical environment, a biological environment or in physiological environment.

In one aspect of the invention use of a compound according to formula I or a salt thereof, is used in ex vivo click-to-release reactions.

Thus, herein is provided use of a compound according to formula I: 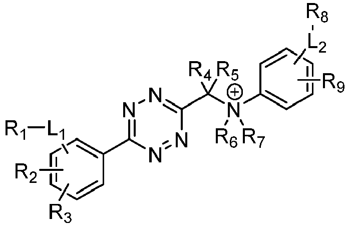 and salts thereof, wherein
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of:

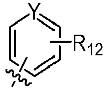

Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity, for use in ex vivo click-to-release reactions, including use wherein the compound of formula I or a salt thereof, is clicked with a dienophile with Diels-Alder activity complementary to the compound of formula I.

In a preferred embodiment, the use of compounds according to formula I and salts thereof is for use wherein the click-to-release reaction releases an aniline comprising R₈ in a chemical environment.

In another preferred embodiment, the use of compounds according to formula I and salts thereof is for use wherein the click-to-release reaction releases an aniline comprising R₈ in an ex vivo biological environment.

In another preferred embodiment, the use of compounds according to formula I and salts thereof is for use wherein the click-to-release reaction releases an aniline comprising R₈ in an ex vivo physiological environment.

### Examples:

### Example 1: Synthesis of a compound according to formula (I)

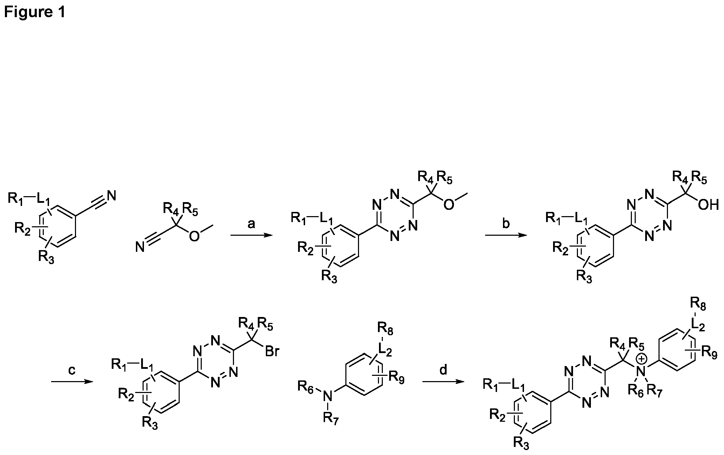

The synthesis of a compound according to formula (I) is shown in Figure 1 and was made subject to the following conditions a) (i) Zn(Otf)₂, H₂NNH₂ , 60 °C, on (ii) NaNOs, AcOH, H₂O, DCM, rt, 20 min (8 - 34%); b) BBr₃, -42 °C, 3 h (37 - 78%); c) NBS, PPh₃, DCM, rt, on (61 - 85%); d) aniline, Et₂O or EtOAc or MeCN, up to 3 day (10 - 49%).

### General Procedure I:

To a mixture of benzonitrile (1.0 equiv.), methoxyacetonitrile (3.0 equiv.) and zinc trifluoromethanesulfonate (0.2 equiv.) was added hydrazine hydrate (10.0 equiv.) dropwise at room temperature. The reaction mixture was heated to 60 °C and stirred overnight. After cooling, the reaction mixture was poured into an aqueous solution of sodium nitrite (20.0 equiv.) and the reaction was diluted with an equal volume of DCM.

Acetic acid (20.0 equiv.) was added dropwise, after a few minutes the solution turned red and is left reaction for an additional 30 min. The mixture was extracted with DCM (3 ×), the combined organic phases were washed with brine and dried over MgSO₄, the obtained crude was a red solid. The crude product was purified by automated column chromatography (silica gel, DCM in heptane 50 - 100% v/v or MeOH in DCM 0 - 5% v/v) yielding the desired product as a pink solid.

### General Procedure II:

The methoxymethyl-Tz (1.0 equiv.) was dissolved in dry DCM and the solution was cooled to -42 °C. Boron tribromide (1 M in DCM) (3.0 equiv.) was added dropwise and the reaction stirred for 3 h. The solution was transferred to an ice bath and after one minute the reaction was quenched with MeOH. The solution turned from deep orange to pink. This reaction was stirred for 5 min at 0°C. H₂O was added and the mixture was extracted with DCM or EtOAc (3 ×). The organic phase was dried over MgSO₄ and the solvent removed under reduced pressure. The crude was purified by automated column chromatography (silica gel, DCM in heptane 50 - 100% v/v or MeOH in DCM 0 - 5% v/v) yielding the desired product as a pink solid.

### General Procedure III:

The hydroxymethyl-Tz (1.0 equiv.) was dissolved in dry DCM and under an argon atmosphere was added to PPh₃ (1.2 equiv.) and *N*-bromosuccinimide (1.2 equiv). The reaction was covered in foil and stirred overnight. H₂O was added, the phases separated, the organic phase washed with H₂O (3 ×) and dried over MgSO₄. The crude was purified by automated column chromatography (silica gel, DCM in heptane 50 - 100% v/v or MeOH in DCM 0 - 5% v/v) yielding the desired product as a pink solid.

### General Procedure IV:

The bromomethyl-Tz (1.0 equiv.) was dissolved in EtOAc, dry MeCN or Et₂O and the corresponding *N*,*N*-dimethylaniline (2.0 equiv) was added in a single portion. The reaction mixture was stirred for 48 hours, at room temperature. Afterwards, the reaction was diluted with Et₂O and the precipitate was filtered and washed extensively with Et₂O. The pink precipitate was dried over high vaccuum.

### 3-(methoxymethyl)-6-(3-methoxyphenyl)-1,2,4,5-tetrazine (1)

The title compound was prepared according to general procedure I, starting from 3-methoxybenzonitile (1.0 g, 7.50 mmol), which yielded a pink solid (589 mg, 2.54 mmol, 34%). ¹H NMR (400 MHz, Chloroform-d) δ 8.23 (ddd, *J* = 7.7, 1.6, 1.0 Hz, 1H), 8.15 (dd, *J* = 2.7, 1.6 Hz, 1H), 7.51 (t, *J* = 8.0 Hz, 1H), 7.18 (ddd, *J* = 8.3, 2.7, 1.0 Hz, 1H), 5.10 (s, 2H), 3.93 (s, 3H), 3.63 (s, 3H); ¹³C NMR (101 MHz, Chloroform-d) δ 166.4, 165.1, 160.5, 132.9, 130.6, 121.0, 119.8, 112.6, 72.4, 59.8, 55.7.

### 3-(4-fluorophenyl)-6-(methoxymethyl)-1,2,4,5-tetrazine (2)

The title compound was prepared according to general procedure I, starting from 4-fluorobenzonitile (200 mg, 1.65 mmol), which yielded a pink solid 364 mg, 0.61 mmol, 37%). ¹H NMR (400 MHz, Chloroform-d) δ 8.71 - 8.63 (m, 2H), 7.35 - 7.26 (m, 2H), 5.10 (s, 2H), 3.64 (s, 3H).

### 3-(methoxymethyl)-6-phenyl-1,2,4,5-tetrazine (3)

The title compound was prepared according to general procedure I, starting from benzonitile (500 mg, 4.85 mmol), which yielded a pink solid (268 mg, 1.33 mmol, 27%). ¹H NMR (400 MHz, Chloroform-d) δ 8.66 - 8.61 (m, 2H), 7.68 - 7.57 (m, 3H), 5.11 (s, 2H), 3.64 (s, 3H). ¹³C NMR (101 MHz, Chloroform-d) δ 166.4, 165.2, 133.1, 131.7, 129.5, 128.4, 72.4, 59.9.

### 3-(methoxymethyl)-6-(p-tolyl)-1,2,4,5-tetrazine (4)

The title compound was prepared according to general procedure I, starting from 4-methylbenzonitile (1.82 g, 25.6 mmol), which yielded a pink solid (512 mg, 2.37 mmol, 27%). ¹H NMR (600 MHz, Chloroform-d) δ 8.51 (dd, *J* = 8.4, 1.8 Hz, 2H), 7.47 - 7.35 (m, 2H), 5.08 (s, 2H), 3.63 (s, 3H), 2.64 - 2.33 (m, 3H). ¹³C NMR (151 MHz, Chloroform-d) δ 166.1, 165.2, 143.9, 130.2, 128.9, 128.4, 72.4, 59.8, 21.9.

### 3-(methoxymethyl)-6-(4-methoxyphenyl)-1,2,4,5-tetrazine (5)

The title compound was prepared according to general procedure I, starting from 4-methoxybenzonitile (1.0 g, 7.50 mmol), which yielded a pink solid (516 mg, 2.22 mmol, 30%). LCMS (ESI) *m*/*z* = 233.1 [M + H]⁺; ¹H NMR (400 MHz, Chloroform-d) δ 8.63 - 8.55 (m, 2H), 7.15 - 7.04 (m, 2H), 5.06 (s, 2H), 3.92 (s, 3H), 3.62 (s, 3H). ¹³C NMR (101 MHz, Chloroform-d) δ 165.7, 164.8, 163.8, 130.3, 124.1, 114.9, 72.4, 59.7, 55.7.

### 4-(6-(methoxymethyl)-1,2,4,5-tetrazin-3-yl)phenol (6)

The title compound was prepared according to general procedure I, starting from 4-hydroxybenzonitile (1.0 g, 8.40 mmol), which yielded a pink solid (138 mg, 0.63 mmol, 8%). LCMS (ESI) *m*/*z* = 219.1 [M + H]⁺; ¹H NMR (600 MHz, Methanol-*d*₄) δ 8.43 (d, *J* = 8.9 Hz, 2H), 6.99 (d, *J* = 8.8 Hz, 2H), 5.00 (s, 2H), 3.57 (s, 3H). ¹³C NMR (151 MHz, Methanol-*d*₄) δ 166.8, 166.1, 163.5, 131.1, 124.0, 117.2, 73.2, 59.6.

### (6-(3-methoxyphenyl)-1,2,4,5-tetrazin-3-yl)methanol (7)

The title compound was prepared according to general procedure II, starting from 1 (100 mg, 0.43 mmol), which yielded a pink solid (35 mg, 0.16 mmol, 37%). LCMS (ESI) *m*/*z* = 219.1 [M + H]⁺; ¹H NMR (600 MHz, Chloroform-d) δ 8.23 (dt, *J* = 7.7, 1.3 Hz, 1H), 8.15 (dd, *J* = 2.7, 1.6 Hz, 1H), 7.52 (t, *J* = 8.0 Hz, 1H), 7.20 (ddd, *J* = 8.3, 2.7, 1.0 Hz, 1H), 5.33 (s, 2H), 3.94 (s, 3H). ¹³C NMR (151 MHz, Chloroform-d) δ 167.5, 165.5, 160.6, 132.8, 130.6, 120.9, 119.9, 112.6, 63.0, 55.7.

### (6-(4-fluorophenyl)-1,2,4,5-tetrazin-3-yl)methanol (8)

The title compound was prepared according to general procedure II, starting from **2** (420 mg, 1.91 mmol), which yielded a pink solid 288 mg, 1.40 mmol, 73%). ¹H NMR (600 MHz, Chloroform-d) δ 8.68 - 8.62 (m, 2H), 7.30 (t, *J* = 8.6 Hz, 2H), 5.33 (s, 2H), 3.09 (brs, 1H). ¹³C NMR (151 MHz, CDCl₃) δ 167.4, 166.2 (d, *J* = 254.9 Hz), 164.8, 130.8 (d, *J* = 9.0 Hz), 127.8 (d, *J* = 3.2 Hz), 116.8 (d, *J* = 22.1 Hz), 63.0. ¹⁹F NMR (376 MHz, CDCl₃) δ -105.6.

### (6-phenyl-1,2,4,5-tetrazin-3-yl)methanol (9)

The title compound was prepared according to general procedure II, starting from 3 (250 mg, 1.24 mmol), which yielded a pink solid (182 mg, 0.97 mmol, 78%). LCMS (ESI) *m*/*z =* 189.1 [M + H]⁺; ¹H NMR (400 MHz, Chloroform-*d*) δ 8.72 - 8.55 (m, 2H), 7.76 - 7.53 (m, 3H), 5.34 (d, *J* = 5.8 Hz, 2H), 3.06 (t, *J* = 6.2 Hz, 1H). ¹³C NMR (101 MHz, Chloroform-d) δ 167.4, 165.6, 133.2, 131.6, 129.5, 128.4, 63.0.

### (6-(p-tolyl)-1,2,4,5-tetrazin-3-yl)methanol (10)

The title compound was prepared according to general procedure II, starting from **4** (216 mg, 1.00 mmol), which yielded a pink solid (120 mg, 0.59 mmol, 59%). ¹H NMR (600 MHz, CDCl₃) δ 8.50 (d, *J* = 8.2 Hz, 2H), 7.41 (d, *J* = 8.2 Hz, 2H), 5.31 (d, *J* = 4.8 Hz, 2H), 3.10 (t, *J* = 4.8 Hz, 1H), 2.48 (s, 3H). ¹³C NMR (151 MHz, CDCl₃) δ 167.2, 165.6, 144.0, 130.3, 128.8, 128.3, 63.0, 21.9.

### (6-(4-methoxyphenyl)-1,2,4,5-tetrazin-3-yl)methanol (11)

The title compound was prepared according to general procedure II, starting from **5** (100 mg, 0.43 mmol), which yielded a pink solid (47 mg, 0.22 mmol, 50%). LCMS (ESI) *m*/*z* = 219.1 [M + H]⁺; ¹H NMR (600 MHz, Chloroform-d) δ 8.58 (d, *J* = 9.1 Hz, 3H), 7.10 (d, *J* = 9.1 Hz, 3H), 5.29 (s, 2H), 3.93 (s, 3H). ¹³C NMR (151 MHz, Chloroform-d) δ 166.8, 165.2, 163.8, 130.3, 124.0, 115.0, 63.0, 55.7.

### 4-(6-(hydroxymethyl)-1,2,4,5-tetrazin-3-yl)phenol (12)

The title compound was prepared according to general procedure II, starting from **6**(130 mg, 0.60 mmol), which yielded a pink solid (94 mg, 0.46 mmol, 77%). LCMS (ESI) *m*/*z* = 205.1 [M + H]⁺; ¹H NMR (600 MHz, Methanol-*d*₄) δ 8.45 (d, *J* = 8.8 Hz, 2H), 7.00 (d, *J* = 8.8 Hz, 2H), 5.13 (s, 2H). ¹³C NMR (151 MHz, Methanol-*d*₄) δ 168.8, 166.1, 163.5, 131.1, 124.2, 117.2, 63.5.

### 3-(bromomethyl)-6-(3-methoxyphenyl)-1,2,4,5-tetrazine (13)

The title compound was prepared according to general procedure III, starting from 7 (35 mg, 0.16 mmol), which yielded a pink solid (29 mg, 0.10 mmol, 64%). ¹H NMR (600 MHz, Chloroform-d) δ 8.24 (dt, *J* = 7.7, 1.2 Hz, 1H), 8.16 (dd, *J* = 2.7, 1.6 Hz, 1H), 7.52 (t, *J* = 8.0 Hz, 1H), 7.20 (ddd, *J* = 8.2, 2.7, 1.0 Hz, 1H), 5.00 (s, 2H), 3.94 (s, 3H). ¹³C NMR (151 MHz, Chloroform-d) δ 167.5, 164.1, 160.6, 132.6, 130.7, 121.2, 120.2, 112.7, 55.7, 27.8.

### 3-(bromomethyl)-6-(4-fluorophenyl)-1,2,4,5-tetrazine (14)

The title compound was prepared according to general procedure III, starting from **8** (31 mg, 0.15 mmol), which yielded a pink solid (29 mg, 0.11 mmol, 71%). ¹H NMR (600 MHz, CDCl₃) δ 8.69 - 8.64 (m, 2H), 7.32 - 7.27 (m, 2H), 4.99 (s, 2H). ¹³C NMR (151 MHz, CDCl₃) δ 167.4, 166.3 (d, *J* = 255.4 Hz), 163.4, 131.0 (d, *J* = 9.3 Hz), 127.6 (d, *J* = 3.0 Hz), 116.9 (d, *J* = 22.1 Hz), 27.7.

### 3-(bromomethyl)-6-phenyl-1,2,4,5-tetrazine (15)

The title compound was prepared according to general procedure I, starting from **9** (50 mg, 0.27 mmol), which yielded a pink solid (57mg, 0.23 mmol, 85%). ¹H NMR (600 MHz, Chloroform-d) δ 8.67 - 8.59 (m, 2H), 7.70 - 7.64 (m, 1H), 7.62 (tt, *J* = 7.6, 1.6 Hz, 2H), 5.00 (s, 2H). ¹³C NMR (151 MHz, Chloroform-d) δ 167.4, 164.3, 133.4, 131.4, 129.6, 128.6, 27.8.

### 3-(bromomethyl)-6-(p-tolyl)-1,2,4,5-tetrazine (16)

The title compound was prepared according to general procedure III, starting from **10** (101 mg, 0.50 mmol), which yielded a pink solid (95 mg, 0.36 mmol, 72%). ¹H NMR (600 MHz, CDCl₃) δ 8.50 (d, *J* = 8.1 Hz, 2H), 7.40 (d, *J* = 7.9 Hz, 2H), 4.98 (s, 2H), 2.47 (s, 3H). ¹³C NMR (151 MHz, CDCl₃) δ 167.1, 164.2, 144.2, 130.3, 128.6, 128.5, 27.9, 21.9.

### 3-(bromomethyl)-6-(4-methoxyphenyl)-1,2,4,5-tetrazine (17)

The title compound was prepared according to general procedure I, starting from **11** (60 mg, 0.28 mmol), which yielded a pink solid (58 mg, 0.21 mmol, 75%). ¹H NMR (600 MHz, Chloroform-d) δ 8.59 (d, *J* = 8.9 Hz, 2H), 7.10 (d, *J* = 8.9 Hz, 2H), 4.97 (s, 2H), 3.93 (s, 3H). ¹³C NMR (151 MHz, Chloroform-d) δ 166.8, 164.0, 163.9, 130.6, 123.7, 115.0, 55.7, 28.0.

### 4-(6-(bromomethyl)-1,2,4,5-tetrazin-3-yl)phenol (18)

The title compound was prepared according to general procedure I, starting from **12** (20 mg, 0.10 mmol), which yielded a pink solid (16 mg, 0.06 mmol, 61%). ¹H NMR (600 MHz, Methanol-*d*₄) δ 8.46 (d, *J* = 8.8 Hz, 2H), 7.00 (d, *J* = 8.9 Hz, 2H), 5.01 (s, 2H). ¹³C NMR (151 MHz, Methanol-*d*₄) δ 168.3, 165.1, 163.8, 131.4, 123.9, 117.3, 28.9.

### N-((6-(3-methoxyphenyl)-1,2,4,5-tetrazin-3-yl)methyl)-N,N-dimethylbenzenaminium bromide (19)

The title compound was prepared according to general procedure IV, starting from **13** (10 mg, 0.037 mmol), which yielded a pink solid (3 mg, 0.008 mmol, 21%). LCMS (ESI) *m*/*z* = 322.2 [M + H]⁺; ¹H NMR (600 MHz, Methanol-*d*₄) δ 8.15 (ddd, *J* = 7.8, 1.6, 0.9 Hz, 1H), 8.08 (dd, *J* = 2.7, 1.6 Hz, 1H), 7.97 - 7.94 (m, 2H), 7.64 - 7.56 (m, 3H), 7.55 (t, *J* = 8.0 Hz, 1H), 7.27 (ddd, *J* = 8.4, 2.7, 1.0 Hz, 1H), 5.82 (s, 2H), 3.97 (s, 6H), 3.90 (s, 3H). ¹³C NMR (151 MHz, Methanol-*d*₄) δ 165.6, 162.9, 162.0, 146.1, 133.7, 132.1, 131.7, 122.2, 122.0, 121.0, 120.7, 114.3, 70.7, 56.03, 55.98.

### N-((6-(4-fluorophenyl)-1,2,4,5-tetrazin-3-yl)methyl)-N,N-dimethylbenzenaminium bromide (20)

The title compound was prepared according to general procedure IV, starting from **14** (20 mg, 0.074 mmol), which yielded a pink solid (19.2 mg, 0.049 mmol, 66%). LCMS (ESI) m/z = 310.2 [M]⁺; ¹H NMR (600 MHz, MeOD) δ 8.65 - 8.59 (m, 2H), 7.98 - 7.93 (m, 2H), 7.68 - 7.55 (m, 3H), 7.42 - 7.35 (m, 2H), 5.83 (s, 2H), 3.98 (s, 6H). ¹³C NMR (151 MHz, MeOD) δ 167.7 (d, *J* = 254.2 Hz), 164.9, 162.8, 146.1, 132.3 (d, *J* = 9.4 Hz), 132.1, 131.7, 129.0 (d, *J*= 3.2 Hz), 122.2, 117.7 (d, *J* = 22.4 Hz), 70.7, 56.0.

### N,N-dimethyl-N-((6-phenyl-1,2,4,5-tetrazin-3-yl)methyl)benzenaminium bromide (21)

The title compound was prepared according to general procedure IV, starting from **15** (22 mg, 0.088 mmol), which yielded a pink solid (16 mg, 0.043 mmol, 49%). LCMS (ESI) *m*/*z* = 292.2 [M + H]⁺; ¹H NMR (400 MHz, Methanol-*d*₄) δ 8.59 - 8.52 (m, 2H), 7.97 - 7.89 (m, 2H), 7.74 - 7.67 (m, 1H), 7.67 - 7.56 (m, 5H), 5.82 (s, 2H), 3.97 (s, 6H). ¹³C NMR (101 MHz, Methanol-*d*₄) δ 165.7, 165.1, 134.7, 132.1, 132.1, 131.7, 130.6, 129.6, 122.2, 56.0, 49.7.

### N,N-dimethyl-N-((6-(p-tolyl)-1,2,4,5-tetrazin-3-yl)methyl)benzenaminium bromide (22)

The title compound was prepared according to general procedure IV, starting from **16** (20 mg, 0.075 mmol), which yielded a pink solid (17.9 mg, 0.046 mmol, 61%). LCMS (ESI) m/z = 306.2 [M]⁺; ¹H NMR (600 MHz, MeOD) δ 8.47 - 8.42 (m, 2H), 7.97 - 7.91 (m, 2H), 7.65 - 7.54 (m, 3H), 7.45 (d, *J* = 7.7 Hz, 2H), 5.80 (s, 2H), 3.97 (s, 6H), 2.47 (s, 3H). ¹³C NMR (151 MHz, MeOD) δ 165.7, 162.6, 146.1, 132.1, 131.7, 131.3, 129.7, 129.6, 122.2, 70.8, 56.0, 21.7. 1.

### N-((6-(4-methoxyphenyl)-1,2,4,5-tetrazin-3-yl)methyl)-N,N-dimethylbenzenaminium bromide (23)

The title compound was prepared according to general procedure IV, starting from **17** (10 mg, 0.036 mmol), which yielded a pink solid (5 mg, 0.012 mmol, 35%). LCMS (ESI) *m*/*z* = 322.2 [M + H]⁺; ¹H NMR (600 MHz, Methanol-*d*₄) δ 8.53 - 8.49 (m, 2H), 7.95 - 7.92 (m, 2H), 7.64 - 7.55 (m, 3H), 7.18 - 7.14 (m, 2H), 5.76 (s, 2H), 3.96 (s, 6H), 3.92 (s, 3H). ¹³C NMR (151 MHz, Methanol-*d*₄) δ 165.9, 165.3, 162.2, 146.0, 132.1, 131.7, 131.6, 124.6, 122.2, 116.1, 70.9, 56.2, 55.9.

### N-((6-(4-hydroxyphenyl)-1,2,4,5-tetrazin-3-yl)methyl)-N,N-dimethylbenzenaminium bromide (24)

The title compound was prepared according to general procedure IV, starting from **18** (20 g, 0.075 mmol), which yielded a pink solid (3 mg, 0.008 mmol, 10%). LCMS (ESI) *m*/*z* = 308.2 [M + H]⁺; ¹H NMR (600 MHz, Methanol-*d*₄) δ 8.43 - 8.40 (m, 2H), 7.94 - 7.90 (m, 2H), 7.63 - 7.55 (m, 3H), 7.01 - 6.96 (m, 2H), 5.73 (s, 2H), 3.95 (s, 6H). ¹³C NMR (151 MHz, Methanol-*d*₄) δ 165.4, 164.5, 161.9, 146.0, 132.1, 131.9, 131.6, 123.2, 122.2, 117.5, 70.9, 55.8.

### N,N,4-trimethyl-N-((6-phenyl-1,2,4,5-tetrazin-3-yl)methyl)benzenaminium bromide (25)

The title compound was prepared according to general procedure IV, starting from **15** (10 mg, 0.040 mmol), which yielded a pink solid (13 mg, 0.033 mmol, 84%). LCMS (ESI) m/z = 306.2 [M]⁺; ¹H NMR (600 MHz, MeOD) δ 8.58 - 8.55 (m, 2H), 7.81 - 7.77 (m, 2H), 7.73 - 7.69 (m, 1H), 7.67 - 7.61 (m, 2H), 7.43 - 7.39 (m, 2H), 5.78 (s, 2H), 3.94 (s, 6H), 2.39 (s, 3H). ¹³C NMR (151 MHz, MeOD) δ 165.7, 162.9, 143.6, 142.9, 134.7, 132.5, 132.0, 130.6, 129.6, 121.9, 70.7, 56.1, 20.8.

### 4-methoxy-N,N-dimethyl-N-((6-phenyl-1,2,4,5-tetrazin-3-yl)methyl)benzenaminium bromide (26)

The title compound was prepared according to general procedure IV, starting from **15** (22 mg, 0.088 mmol), which yielded a pink solid (11 mg, 0.088 mmol, 36%). LCMS (ESI) *m*/*z* = 322.2 [M + H]⁺; ¹H NMR (600 MHz, Methanol-*d*₄) δ 8.59 - 8.55 (m, 2H), 7.85 - 7.80 (m, 2H), 7.73 - 7.69 (m, 1H), 7.66 - 7.61 (m, 2H), 7.10 - 7.02 (m, 2H), 5.76 (s, 2H), 3.93 (s, 6H), 3.84 (s, 3H). ¹³C NMR (151 MHz, Methanol-*d*₄) δ 165.7, 162.9, 162.4, 138.4, 134.7, 132.6, 130.6, 129.6, 123.7, 116.2, 71.0, 56.3, 56.2.

### 4-hydroxy-N,N-dimethyl-N-((6-phenyl-1,2,4,5-tetrazin-3-yl)methyl)benzenaminium bromide (27)

The title compound was prepared according to general procedure IV, starting from **15** (30 mg, 0.088 mmol), which yielded a pink solid (8 mg, 0.040 mmol, 20%). LCMS (ESI) *m*/*z* = 308.2 [M + H]⁺; ¹H NMR (600 MHz, Methanol-*d*₄) δ 8.61 - 8.54 (m, 2H), 7.75 - 7.68 (m, 3H), 7.64 (t, *J* = 7.7 Hz, 2H), 6.93 - 6.87 (m, 2H), 5.73 (s, 2H), 3.90 (s, 6H). ¹³C NMR (151 MHz, Methanol-*d*₄) δ 165.7, 162.9, 160.6, 137.3, 134.7, 132.6, 130.6, 129.6, 123.6, 117.5, 71.0, 56.1.

### 3-(iodomethyl)-6-(4-methoxyphenyl)-1,2,4,5-tetrazine (28)

**11** (200.0 mg, 0.92 mmol, 1.0 equiv.) was dissolved in 5 mL of dry DCM and under an argon atmosphere was added to PPh₃ (264.4 mg, 1.01 mmol, 1.1 equiv.) and *N-*iodosuccinimide (226.8 mg, 1.01 mmol, 1.1 equiv). The reaction was covered in foil and stirred overnight. 5 mL of H₂O was added, the phases separated, the organic phase washed with H₂O (3 × 5 mL) and dried over MgSO₄. The crude was purified by automated column chromatography (silica gel, DCM in heptane 50 - 100% v/v or MeOH in DCM 0 - 5% v/v) yielding the desired product as a pink solid (179.3 mg, 0.55 mmol, 60%). ¹H NMR (600 MHz, CDCl₃) δ 8.58 (d, *J* = 9.0 Hz, 2H), 7.17 - 7.04 (m, 2H), 4.93 (s, 2H), 3.93 (s, 3H). ¹³C NMR (151 MHz, CDCl₃) δ 168.7, 163.9, 163.3, 130.4, 123.9, 115.0, 115.0, 55.7.

### 4-methoxy-N-((6-(4-methoxyphenyl)-1,2,4,5-tetrazin-3-yl)methyl)-N,N-dimethylbenzenaminium bromide (29)

The title compound was prepared according to general procedure IV, starting from **28** (32.8 mg, 0.10 mmol), which yielded a pink solid (23.8 mg, 0.050 mmol, 50%). LCMS (ESI) m/z = 352.2 [M]⁺; ¹H NMR (400 MHz, MeOD) δ 8.58 - 8.48 (m, 2H), 7.85 - 7.76 (m, 2H), 7.20 - 7.12 (m, 2H), 7.10 - 7.01 (m, 2H), 5.70 (s, 2H), 3.93 (s, 3H), 3.91 (s, 6H), 3.84 (s, 3H).

### Example 2: Click kinetics of various tetrazines of formula (I)

Nine tetrazines of formula (I) (Table 1) were clicked with equatorial TCO-PEG₄ (*rel-*(1*R*-4*E*-p*R*)-cyclooct-4-en-1-yl (2-(2-(2-(2-hydroxyethoxy)ethoxy)ethoxy)ethyl)carbamate).

Stopped-flow measurements were performed using an SX20-LED stopped-flow spectrophotometer (Applied Photophysics) equipped with a 535 nm LED (optical pathlength 10 mm, full width half-maximum 34 nm) to monitor the characteristic tetrazine visible light absorbance (520-540 nm).

A stock solution of eqTCO-PEG₄ in DMSO was prepared at an approximate concentration above 100 mM. The exact concentration was determined by absorbance titration with 3,6-di-2-pyridyl-1,2,4,5-tetrazine. Stock solutions of tetrazines in DMSO were prepared at a concentration of 10 mM. These initial stock solutions were diluted into PBS (10mM) before stopped-flow analysis to reach a final TCO concentration of 1 mM and a tetrazine concentration of 100 µM. The reagent syringes were loaded with solutions of the Tz and eqTCO-PEG₄, and the instrument was primed. Subsequent data were collected in triplicate to sextuplicate for each tetrazine. Reactions were conducted at 37 °C and recorded automatically at the time of acquisition. Data sets were analyzed by fitting an exponential decay using Prism 6 (Graphpad) to calculate the observed pseudo-first-order rate constants that were converted into second-order rate constants by dividing with the concentration of the excess TCO compound.

**Table 1: second rate order constant (M⁻¹s⁻¹) for the ligation between nine tetrazines and equatorial TCO-PEG₄ (rel-(1R-4E-pR)-cyclooct-4-en-1-yl (2-(2-(2-(2-hydroxyethoxy)ethoxy)ethoxy)ethyl)carbamate).**

| **Entry** | **Compound** | **second order rate constant (M⁻¹ s⁻¹)** |
|---|---|---|
| **1** | **19** | 790 |
| **2** | **20** | 580 |
| **3** | **21** | 650 |
| **4** | **22** | 480 |
| **5** | **23** | 370 |
| **6** | **25** | 620 |
| **7** | **26** | 670 |
| **8** | **27** | 620 |
| **9** | **29** | 340 |

### Example 3: Release kinetics of various tetrazines of formula (I)

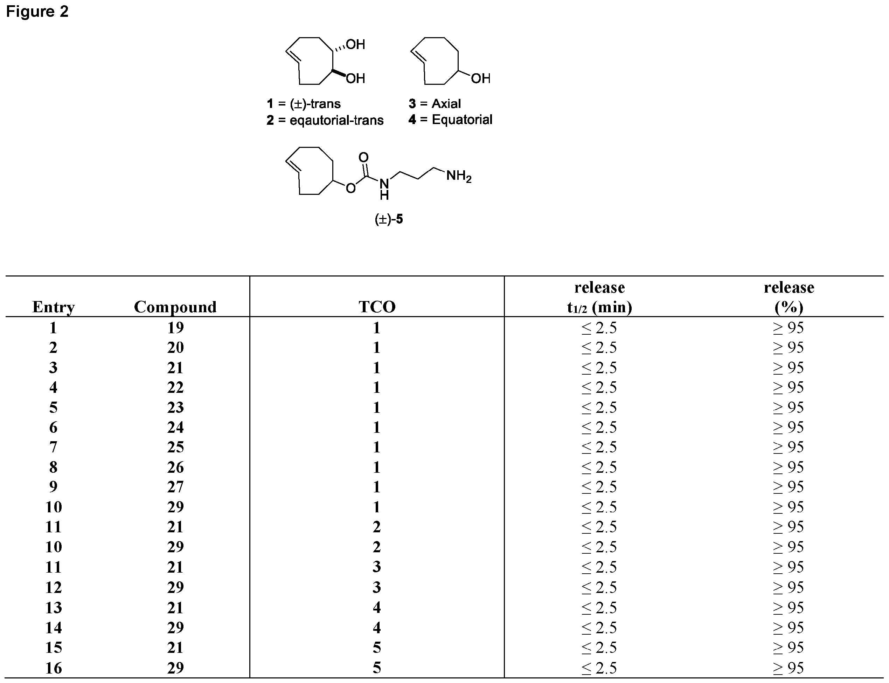

Ten tetrazines of formula (I) (Figure 2) were tested in relation to release kinetics of the various R₁ and R₂ group after click-reaction with five different dienophiles (Figure 2).

The results are shown in Figure 2 for the release kinetics were measured by HPLC at RT, in 9:1 PBS/MeCN solution, using 1.0-1.5 mM Tz and 2 equiv. of TCO derivative (a); based on average peak intensities of the corresponding Tz and corresponding pyridazines, at 254 nm. The release percentage as determined by ¹H-NMR measured in MeOD in a separate experiment.

As shown in in Figure 2, for all tetrazines tested, more than 95% of the compounds in each sample tested had released the anilinium payload with a t½ of less than 2.5 minutes.

### Example 4: Stability tests of various tetrazines of formula (I)

Ten tetrazines of formula (I) (Table 2) were tested in relation to chemical stability in PBS.

The results are shown in Table 2 for the chemical half-life was measured by HPLC at RT, in 9:1 PBS/MeCN solution, using 1.0-1.5 mM Tz (a); based on average peak intensities of the corresponding Tz and the corresponding oxadiazoles (the degradation product), at 254 nm.

As shown in Table 2, for all tetrazines tested, the compounds are of sufficient stability.

**Table 2: chemical stability t½ (min) in PBS of various tetrazines.**

| **Entry** | **Compound** | **PBS stability t_{1/2} (min)** |
|---|---|---|
| **1** | **19** | 48.2 ± 0.5 |
| **2** | **20** | 47.8 ± 3.5 |
| **3** | **21** | 58.0 ± 0.5 |
| **4** | **22** | 107.0 ± 1.0 |
| **5** | **23** | 154.5 ± 41.0 |
| **6** | **24** | 296.8 ± 6.0 |
| **7** | **25** | 68.8 ± 6.0 |
| **8** | **26** | 112.0 ± 21.4 |
| **9** | **27** | 311.2 ± 69.0 |
| **10** | **29** | 561.9 ± 0.75 |

## Claims

1. Compound of formula I: 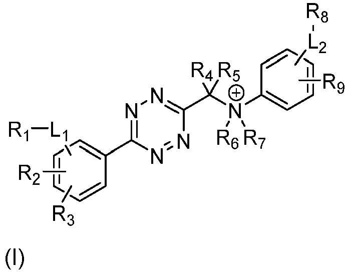 and salts thereof, wherein:
L₁ and L₂ are linkers independently selected from the group consisting of: L₃-(CH₂)ₙ, L₃(CH₂CH₂)ₙO-, -L₃(CH₂)ₙNH -, -L₃(CH₂)ₙCONH -, and -L₃(CH₂)ₙNHCO-, wherein L₃ is -(CH₂)ₘ- or -(CH₂CH₂O)ₘ-, and wherein n and m are integers independently selected from 0-25;
R₁ is a targeting vector, or a substituent selected from: H, Methyl, Ethyl, Propyl, COOH, CONH₂, CONHCH₃, NH₂, NHCH₃, or N(CH₃)₂;
R₂, R₃, and R₉ are independently selected from the group consisting of: H, F, Cl, Br, I, CF₃, NO₂, CH₃, CH₂R₁₀, OH, OR₁₀, NH₂, NHR₁₀, NR₁₀R₁₁, COOH, CONH₂, CONHR₁₀, and CONR₁₀R₁₁, wherein R₁₀ and R₁₁ are independently selected from: -(CH₂)ₘH-, or -(CH₂CH₂O)ₘH-, and wherein m is an integer from 1-25;
R₄ and R₅ are independently selected from: H, Methyl, Ethyl, CF₃, COOH, C₁-C₁₀, CONH₂, or Ar₁
wherein Ar₁ is selected from the group consisting of: 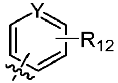
Wherein Y is -CH or N, and wherein R₁₂ is H, F, Cl, Br, I, CF₃, NO₂, OH or C₁-C₅ alkyl;
R₆ and R₇ are independently selected from: H, Methyl, or Ethyl;
R₈ is a payload entity.

2. Compound according to claim 1 as a salt, wherein the salt is a pharmaceutically acceptable salt.

3. Compound according to any of the previous claims, wherein R₁ is a targeting entity selected from: an antibody or a fragment thereof, a nanobody, a polymer, a nanomedicine, a cell, a protein, a peptide, a carbohydrate, a monosaccharide, a polysaccharide, an oligonucleotide, or a small molecule.

4. Compound according to any of the previous claims, wherein R₈ is a therapeutic compound, a diagnostic compound, or a prodrug.

5. Compound according to claim 4, wherein R₈ is a chemotherapeutic agent, an anti-angiogenic agent, a cytotoxic agent, an immunomodulatory agent, a hormone, an antibiotic, an anti-viral agent, an anti-inflammatory agent, or a cell-cycle suppressor.

6. Compound according to claim 4, wherein R₈ is selected from: a radionuclide, an auristatin, a maytansine, a maytansinoid, a calicheamicin, a dactinomycine, a duocarmycin, camptothecin, tubulysin M, a cryptophycin, a pyrrolobenzodiazepine, methotrexate, methopterin, dichloromethotrexate, 5-fluorouracil, a DNA minor groove binder, 6-mercaptopurine, cytosine arabinoside, melphalan, leurosine, leurosideine, actinomycin, an anthracycline, a mitomycin, aminopterin, tallysomycin, podophyllotoxin, vinblastine, vincristine, vindesine, taxol, taxotere retinoic acid, butyric acid, NS-acetyl spermidine, staurosporin, colchicine, camptothecin, esperamicin, and an ene-diyne.

7. Compound according to any of the previous claims, wherein L₃ within at least one of L₁ and L₂ is independently selected from -(CH₂CH₂O)ₘ-, where m is an integer from 1-10; and wherein R₂, R₃ and R₉ are independently selected from: methyl, ethyl, OH, OCH₃, or OCH₂CH₃; and wherein R₄ and R₅ are independently selected from: H, Methyl, Ethyl, or Phenyl.

8. A kit comprising a compound of formula I according to any of the previous claims, and
a dienophile with Diels-Alder activity complementary to the compound of formula I, with the proviso that when R₁ in the compound of formula I is a substituent selected from H, Methyl, Ethyl, Propyl, COOH, CONH₂, CONHCH₃, NH₂, NHCH₃, N(CH₃)₂, then the dienophile with Diels-Alder activity complementary to the compound of formula I comprises a targeting entity.

9. A kit according to claim 8, wherein the dienophile with Diels-Alder activity complementary to compound I has a structure according to Formula II 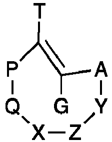
wherein A,Y, Z, X, Q, P each independently are selected from the group consisting of CR^{a}2, C=CR^{a}2, C=O, S, S=O, SO₂, O, NR^{b} and SiR^{c}₂, provided that A and P are not O or S and that NR^{b} if present in A or P, is part of an amide moiety, with at most three of A,Y, Z, X, Q and P being selected from the group consisting of C=CR^{a}₂ and C=O, wherein two or more R moieties together may form a ring or rings, and with the proviso that no sets consisting of adjacent atoms are present selected from the group consisting of O-O, O-NR^{b}, S-NR^{b} O-S, O-S(O), O-S(O)₂, and S-S, and such that Si is only adjacent to CR^{a}₂ or O;
T, G each independently denotes H, or a substituent selected from the group consisting of alkyl, OR', NR'R', F, Cl, Br, or I, with R' being alkyl or aryl;
each R^{a} as above-indicated can independently be selected from the group consisting of H, (hetero)alkyl, aryl, heterocycle, carbocycle, F, Cl, Br, I, CF₃, CF₂-R', NO₂, OR', SR', CN, C(=O)R', S(=O)R', S(=O)₂R", S(=O)₂OR', OS(=O)₂R", PO₃R'₂, Si-R"₃, Si-O-R"₃, B(OR')₂,S(=O)₂NR'₂, NR'S(=O)₂R", C(=O)NR'₂, C(=S)NR'₂, C(=NR')NR'₂,NR'₂, NR"₃⁺,NR'C(=O)R', NR'C(=S)R', NR'C(=O)OR', NR'C(=S)OR', NR'C(=O)SR', NR'C(=S)SR',NR'C(=O)NR'₂, NR'C(=S)NR' ₂, wherein each R' is independently selected from the group consisting of H, (hetero)alkyl, aryl, carbocycle, heterocycle, and each R" is independently selected from the group consisting of (hetero)alkyl, aryl, carbocycle, heterocycle, wherein all (hetero)alkyl, aryl, carbocycle, heterocycle moieties comprised in R^{a} can be unsubstituted or substituted, and wherein two R' or R" groups can together form a ring;
each R^{b} is independently selected from the group consisting of H, (hetero)alkyl, aryl, heterocycle, carbocycle, OR', C(=O)R', C(=O)O-R', C(=O)S-R', C(=S)O-R', C(=S)S-R', C(=O)NR'₂, C(=S)NR'₂, C(=NR')NR'₂, NR'₂, preferably such that no sets consisting of adjacent atoms are present selected from the group consisting of -N-N-, -N-O-, -N-S-, -N-Si- and -N-P-, and wherein each R' is independently selected from the group consisting of H, (hetero)alkyl, aryl, carbocycle, heterocycle, wherein all (hetero)alkyl, aryl, carbocycle, heterocycle moieties comprised in R^{b} can be unsubstituted or substituted, and wherein two R' groups can together form a ring;
each R^{c} as above indicated is independently selected from the group consisting of H, alkyl, aryl, O-alkyl, O-aryl, OH;
wherein two or more R^{a,b,c} moieties together may form a ring;
wherein, optionally, one or more R^{a,b,c} moieties may be bound to one or more of the group consisting of Targeting vector T^{T}, Spacer S^{P}, chelator, albumin-binding moiety, polymersome, micelle, particle, resin, gel, surface, bead, membrane translocation moiety, radioisotope, and fluorescent moiety; wherein, optionally said Targeting agent T^{T}, Spacer S^{P}, chelator, albumin-binding moiety, polymersome, micelle, particle, resin, gel, surface, bead, membrane translocation moiety, radioisotope, and fluorescent moiety, is bound to more than one dienophile moiety.

10. Compound of formula I and pharmaceutically acceptable salts thereof according to any of claims 1 to 7, for use as a medicament.

11. Compound of formula I and pharmaceutically acceptable salts thereof according to any of claims 1 to 7, wherein R₈ is an anti-cancer agent for use in the treatment of cancer.

12. Use of a compound according to any of claims 1 to 7 in ex vivo click-to-release reactions.

13. Use according to claim 12, wherein the click-to-release reaction releases an aniline comprising R₈ in a chemical environment, an ex vivo biological environment or ex vivo physiological environment.
